# EUROPÄISCHE PATENTSCHRIFT

(11) **EP 3 576 795 B1**
(45) Veröffentlichungstag und Bekanntmachung des Hinweises auf die Patenterteilung: **07.04.2021**
(21) Anmeldenummer: 18706422.5
(22) Anmeldetag: 02.02.2018
(51) Int. Cl.: A61K 47/18, A61K 47/20, A61K 9/20, A61K 31/198

(54) **ORALES SCHILDDRÜSENTHERAPEUTIKUM**
ORAL THYROID THERAPEUTIC AGENT
MÉDICAMENT PAR VOIE ORALE POUR LA THYROÏDE

(30) Priorität: 03.02.2017 DE 102017102192; 29.09.2017 DE 102017122807
(43) Veröffentlichungstag der Anmeldung: 11.12.2019
(73) Patentinhaber: Berlin-Chemie AG, 12489 Berlin (DE)
(72) Erfinder: BECKER, Achim, 14163 Berlin (DE)
(74) Vertreter: Dr. Klemens Schubert Patentanwalt
(86) Internationale Anmeldenummer: PCT/EP2018/052713
(87) Internationale Veröffentlichungsnummer: WO 2018/141938

(56) Entgegenhaltungen:
- DE-A1- 19 541 128
- Maísa Teodoro Celestino ET AL: "Rational use of antioxidants in solid oral pharmaceutical preparations", Brazilian Journal of Pharmaceutical Sciences, 1. September 2012 (2012-09-01), Seiten 405-415, XP055469120, DOI: 10.1590/S1984-82502012000300007 Gefunden im Internet: URL:http://www.scielo.br/pdf/bjps/v48n3/a0 7v48n3.pdf

## Beschreibung

Die vorliegende Erfindung betrifft ein orales Schilddrüsentherapeutikum, enthaltend Levothyroxin und/oder Liothyronin als Wirkstoff, mit verbesserter Wirkstoffstabilität.

Levothyroxin (C₁₅H₁₁I₄NO₄, Mᵣ = 776.9 g/mol) entspricht dem als Thyroxin oder T4 bezeichnetem körpereigenem Schilddrüsenhormon und ist ein Freiname (INN). Levothyroxin ist das tetraiodierte Derivat der Aminosäure Tyrosin. In Arzneimitteln wird vorwiegend das Natriumsalz des Levothyroxin verwendet, welches als L-Thyroxin Na oder Levothyroxin-Natrium bezeichnet wird. Levothyroxin ist ferner eine Vorstufe des Triiodthyronin oder T3, welches auch als Liothyronin bezeichnet wird.

Der Wirkstoff Thyroxin und auch dessen Enantiomere in reiner Form, weisen keine ausreichende Stabilität auf, um diese enthaltende orale Pharmazeutika über einen längeren Zeitraum zu lagern. Es ist daher erforderlich, galenische Zubereitungen zu schaffen, welche eine ausreichende Stabilität des Wirkstoffs und der gesamten Zubereitung, in möglichst allen Klimazonen, sicherstellen.

Ein Lösungsansatz hierzu ist die Verwendung spezieller Verpackungen, welche Luftsauerstoff binden oder diesen von der Zubereitung fernhalten, welche den Wirkstoff Levothyroxin enthält. Derartige Vorrichtungen sind beispielsweise in der US 2009/0297566 A1 oder der EP 1 772 395 A1 beschrieben.

Im Stand der Technik sind eine Reihe von Levothyroxinzubereitungen bekannt, welche eine verbesserte Stabilität aufweisen. Hierzu werden den Zubereitungen Stabilisatoren zugesetzt. In der WO 2003/041699 A1, der DE 195 41 128 A1 und der US 8,779,000 B1 wird der Zusatz von Antioxidationsmitteln vorgeschlagen.

Es gibt aber auch Zusammensetzungen, welche bestimmte, sonst übliche Bestandteile nicht enthalten, weil diese die Stabilität des Levothyroxin negativ beeinflussen. Diese Zusammensetzungen sind beispielsweise beschrieben in der US 8,779,000 B1 oder der DE 198 30 246 A1. In der DE 198 30 246 A1 wird explizit der Ausschluss von Antioxidationsmitteln vorgeschlagen.

Bekannt sind ferner Zubereitungen, bei denen der Wirkstoff Levothyroxin mittels bestimmter Hilfsstoffe verkapselt oder eingebettet wird. Derartige Zubereitungen sind in der WO 1998/18610 A1 beschrieben.

Bekannt ist ferner der Zusatz von lod enthaltenden Verbindungen wie Kaliumiodid, zum Stabilisieren der Zusammensetzung.

Im Stand der Technik ist ferner bekannt, dass in vielen festen und flüssigen Arzneimittelzubereitungen schwefelhaltige Stabilisierungsmittel verwendet werden. Dies wird beispielsweise beschrieben in Celestino M.T. [et al.] (Rational use of antioxidants in solid oral pharmaceutical preparations. Brazilian Journal of Pharmaceutical Sciences, Vol. 48, Sep 2012, No. 3, pages 405-415. DOI: 10.1590/S1984-82502012000300007).

Das Problem der Stabilität oraler Schilddrüsentherapeutika konnte jedoch bisher noch nicht zufriedenstellend gelöst werden. Somit besteht ein andauernder Bedarf an stabilen oralen Zubereitungsformen von Schilddrüsentherapeutika mit dem Wirkstoff Levothyroxin und/oder dem Wirkstoff Liothyronin.

Aufgabe der vorliegenden Erfindung ist es daher, die Stabilität von Levothyroxin und/oder Liothyronin enthaltenden oralen Darreichungsformen zu verbessern. Aufgabe der vorliegenden Erfindung ist es insbesondere, eine orale Darreichungsform bereitzustellen, welche in der Klimazone II (25 °C, 60 % relative Feuchte) und der Klimazone IVB (30 °C, 75 % relative Feuchte) die Stabilitätsanforderungen für Fertigarzneimittel erfüllt.

Die Aufgabe wird durch Bereitstellung eines oralen Schilddrüsentherapeutikums mit den Merkmalen des Hauptanspruchs gelöst. Vorteilhafte Ausgestaltungen des erfindungsgemäßen oralen Schilddrüsentherapeutikums sind in den abhängigen Unteransprüchen gekennzeichnet.

Gegenstand der vorliegenden Erfindung ist ein orales Schilddrüsentherapeutikum, enthaltend mindestens einen Wirkstoff in Form eines Schilddrüsenhormons oder dessen pharmazeutisch verträgliche Salze als Wirkstoff, in Kombination mit mindestens einem Antioxidationsmittel und mindestens einem Adsorptionsmittel, wobei das Verhältnis in Gew.-% von Wirkstoff: Antioxidationsmittel : Adsorptionsmittel 1 : 1: 5 bis 1 : 50 : 100 beträgt, wobei das Antioxidationsmittel aus schwefelhaltigen, organischen Antioxidationsmitteln ausgewählt ist, die ausgewählt sind aus Cystein, Cystin, Glutathion, Dimercaptopropansulfonsäure sowie deren Salze und aus Mischungen der vorgenannten Mittel.

Bei der Berechnung des Verhältnisses ist davon auszugehen, dass das erfindungsgemäße orale Schilddrüsentherapeutikum neben dem Wirkstoff, dem Antioxidationsmittel und dem Adsorptionsmittel zusammen mit weiteren pharmazeutisch verträglichen Träger- und Hilfsstoffen insgesamt 100 Gew.-% umfasst.

Eine weitere vorteilhafte Ausgestaltung des erfindungsgemäßen oralen Schilddrüsentherapeutikums ist dadurch gekennzeichnet, dass der Wirkstoff in Form des Schilddrüsenhormons Levothyroxin oder dessen pharmazeutisch verträgliche Salze oder Liothyronin oder dessen pharmazeutisch verträgliche Salze ist, oder dass Mischungen aus den genannten Wirkstoffen enthalten sind.

Weiterhin ist ferner ein orales Schilddrüsentherapeutikum bevorzugt, bei welchem ein weiterer Wirkstoff enthalten ist, wobei der weitere Wirkstoff bevorzugt mindestens ein lodsalz ist, ausgewählt aus Iodiden oder Iodaten der Alkalimetalle, insbesondere Kaliumiodid oder Kaliumiodat, sowie Mischungen der genannten lodsalze.

Erfindungsgemäß bevorzugt ist ein orales Schilddrüsentherapeutikum, wobei weiterhin pharmazeutisch annehmbare Hilfsstoffe, Trägerstoffe und/oder Verdünnungsstoffe enthalten sind.

Ganz besonders bevorzugt ist es, dass das schwefelhaltige, organische Antioxidationsmittel Cystein ist.

Erfindungsgemäß bevorzugt ist auch ein orales Schilddrüsentherapeutikum, wobei das Adsorptionsmittel aus anorganischen Adsorptionsmitteln ausgewählt ist. Bevorzugt sind dabei anorganische Adsorptionsmittel, die basisch oder oxydisch sind.
Besonders bevorzugt ist dabei, dass die anorganischen Adsorptionsmittel ausgewählt sind aus Kieselgelen, Zeolithen, Erdalkalimetalloxiden, Alkalicarbonaten, Alkalihydrogencarbonaten, Erdalkalicarbonaten, Erdalkalihydrogencarbonaten, Erdalkalisilikaten sowie aus Mischungen der vorgenannten Adsorptionsmittel.
Ganz besonders bevorzugt ist es, dass das Erdalkalimetalloxid Magnesiumoxid und/oder das Erdalkalisilikat Magnesiumsilikathydrat (Talkum) und/oder das Alkalicarbonat Natriumcarbonat ist.

Bevorzugt ist ferner ein orales Schilddrüsentherapeutikum, wobei das Verhältnis in Gew.-% von Wirkstoff: Antioxidationsmittel: Adsorptionsmittel 1 : 5 : 5 bis 1 : 10 : 40 beträgt.

Erfindungsgemäß bevorzugt ist ferner ein orales Schilddrüsentherapeutikum, wobei der Wirkstoffgehalt 0,01 bis 0,5 Gew.-% beträgt.

Bevorzugt ist auch ein orales Schilddrüsentherapeutikum, wobei der Wirkstoffgehalt einer Einzeldosierung 25 µg bis 300 µg beträgt. Ganz besonders bevorzugt ist ein Wirkstoffgehalt von 25 µg bis 150 µg.

Erfindungsgemäß bevorzugt ist es weiterhin, dass der Gehalt an Cystein 0,1 - 5 Gew.-% beträgt.

Erfindungsgemäß bevorzugt ist es, dass der Gehalt an MgO 0,5 - 10 Gew.-% beträgt. Magnesiumoxid kann flüchtige Verbindungen durch Ausbildung von Van-der-Waals-Bindungen adsorbieren.

Erfindungsgemäß bevorzugt ist es, dass die Zubereitung zusätzlich prägelatinierte Stärke enthält. Besonders bevorzugt sind hierbei Anteile von 30 - 60 Gew.-%.

Weiterhin ist es erfindungsgemäß bevorzugt, dass mikrokristalline Cellulose enthalten ist. Besonders bevorzugt sind hierbei Anteile von 10 - 30 Gew.-%.

Zudem ist es erfindungsgemäß bevorzugt, dass Maisstärke enthalten ist. Besonders bevorzugt sind hierbei Anteile von 10 - 50 Gew.-%.

Bevorzugte orale Schilddrüsentherapeutika sind dadurch gekennzeichnet, dass der Wirkstoffgehalt 0,01 - 1 Gew.-% beträgt. Dabei ist es besonders bevorzugt, dass der Wirkstoffgehalt 0,1 - 0,5 Gew.-% beträgt.

Dies bedeutet dass ein erfindungsgemäßes orales Schilddrüsentherapeutikum die folgende Zusammensetzung in Gew.-% aufweist:

| | |
|---|---|
| Levothyroxin-Natrium | 0,01 - 1% |
| weitere Wirkstoffe | 0 - 10% |
| Antioxidationsmittel | 0,01 - 50% |
| Adsorptionsmittel | 0,05 - 99% |
| Tablettierhilfsstoffe | ad 100% |

Besonders bevorzugt sind erfindungsgemäße orale Schilddrüsentherapeutika, welche die folgende Zusammensetzung in Gew.-% aufweisen:

| | |
|---|---|
| Levothyroxin-Natrium | 0,1 - 0,5% |
| weitere Wirkstoffe | 0 - 10% |
| Antioxidationsmittel | 0,5 - 5% |
| Adsorptionsmittel | 0,5 - 40% |
| Tablettierhilfsstoffe | ad 100% |

Weiterhin bevorzugt sind erfindungsgemäße orale Schilddrüsentherapeutika, welche die folgende Zusammensetzung in Gew.-% aufweisen:

| | |
|---|---|
| Liothyronin-Natrium | 0,01 - 1% |
| weitere Wirkstoffe | 0 - 10% |
| Antioxidationsmittel | 0,01 - 50% |
| Adsorptionsmittel | 0,05 - 99% |
| Tablettierhilfsstoffe | ad 100% |

Bevorzugt sind auch erfindungsgemäße orale Schilddrüsentherapeutika, welche die folgende Zusammensetzung in Gew.-% aufweisen:

| | |
|---|---|
| Levothyroxin-Natrium | 0,1 - 0,5% |
| Liothyronin-Natrium | 0,01 - 0,5% |
| Antioxidationsmittel | 0,5 - 5% |
| Adsorptionsmittel | 0,5 - 40% |
| Tablettierhilfsstoffe | ad 100% |

Bevorzugt sind ferner erfindungsgemäße orale Schilddrüsentherapeutika, welche die folgende Zusammensetzung in Gew.-% aufweisen:

| | |
|---|---|
| Levothyroxin-Natrium | 0,1 - 0,5% |
| lodsalze | 0,01 - 5% |
| Antioxidationsmittel | 0,5 - 5% |
| Adsorptionsmittel | 0,5 - 40% |
| Tablettierhilfsstoffe | ad 100% |

Überraschenderweise wurde gefunden, dass das Antioxidationsmittel und das Adsorptionsmittel in einem hohen stöchiometrischen Überschuss bezogen auf den Wirkstoff in der erfindungsgemäßen Zubereitung vorhanden sein müssen, um die gewünschte Stabilität des Wirkstoffs zu erreichen. Es wurde gefunden, dass vorteilhafterweise ein 10- bis 50-facher stöchiometrischer Überschuss an Antioxidationsmittel, bezogen auf den Wirkstoff, zu wählen ist. Entsprechendes gilt auch für das Adsorptionsmittel. Auch hier ist ein 10-bis 50-facher stöchiometrischer Überschuss, bezogen auf den Wirkstoff, vorteilhaft.

Erfindungsgemäß bevorzugt ist es auch, dass das orale Schilddrüsentherapeutikum in Form einer Basiszubereitung in Form von einfach oder mehrfach teilbaren Tabletten vorliegt. Besonders bevorzugt sind hierbei teilbare Tabletten, die eine Bruchrille, mehrere parallel zueinander angeordnete Bruchrillen oder eine Kreuzbruchkerbe aufweisen. Damit ist es möglich, individuelle Dosierungen bei Patienten zu erzielen, ohne dass für jede Dosierung ein separates Arzneimittel bereit gehalten oder hergestellt werden muss.

### Kurze Beschreibung der Zeichnungen

FIG. 1 zeigt die Versuchsergebnisse des Beispiels 4, nämlich die Ergebnisse der Stabilitätsuntersuchungen der erfindungsgemäßen oralen Schilddrüsentherapeutika.
FIG. 2 zeigt das Freisetzungsprofil der erfindungsgemäßen Zubereitung gemäß Beispiel 3, nämlich die Ergebnisse der Ermittlung der Freisetzungsrate.

Die vorliegende Erfindung betrifft somit ein Konzept und Verfahren zur Herstellung eines Schilddrüsentherapeutikums in verschiedenen Dosierungsstärken aus einem Grund- bzw. Basisgranulat, welches aus der Basiszubereitung hergestellt wird, um eine in Abhängigkeit von der gegebenen Dosis lineare Pharmakokinetik für das enthaltene Schilddrüsenhormon nach dessen oraler Verabreichung zu erhalten.

Die Arzneimittel der Erfindung werden mit den üblichen festen oder flüssigen Hilfs-, Träger- und/oder Verdünnungsstoffen und den üblicherweise eingesetzten Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Lösungen, Suspensionen, Tabletten, Filmtabletten, Kapseln oder spezielle Retard-Arzneiformen.

Entsprechende Retardformen können durch Mischen des Wirkstoffs, zusammen mit dem Antioxidationsmittel und dem Adsorptionsmittel in der erfindungsgemäßen Kombination, mit bekannten Hilfsstoffen oder Mitteln zur Erzielung eines Depoteffektes hergestellt werden. Die Tabletten können auch aus mehreren Schichten bestehen.

Entsprechend können auch Mikrotabletten hergestellt werden, welche dann in Kapseln eingefüllt werden können. Aber auch das Pulver/Granulat der Basisrezeptur kann direkt in Kapseln abgefüllt werden. Mit Hilfe üblicher Kapselfüllmaschinen ist es daher möglich, verschieden dosierte Präparate ohne große Umrüstzeiten herzustellen, da stets die identische Basisrezeptur verwendet wird. Es ist lediglich eine Einstellung der Füllmenge erforderlich, um anders dosierte Formen herzustellen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die dem Fachmann bekannten Hilfsstoffe verwendet werden können.

Die Herstellung der erfindungsgemäßen oralen pharmazeutischen Zubereitungen ist an sich bekannt und in dem Fachmann bekannten Handbüchern beschrieben (z.B. Hager, Hagers Handbuch der Pharmazeutischen Praxis, 5. Auflage, Springer Verlag, 1999, List et al., Arzneiformenlehre, 2. Auflage, Wissenschaftliche Verlagsgesellschaft 1985, Bauer et al., Pharmazeutische Technologie, 9. Auflage, Wissenschaftliche Verlagsgesellschaft mbH, 2012).

Das erfindungsgemäße orale Schilddrüsentherapeutikum weist eine hohe Stabilität in allen Klimazonen auf. Die Lagerstabilität gegenüber herkömmlichen Zubereitungen ist wesentlich verbessert. Damit ist es möglich, eine Zubereitung für alle Klimazonen bereitzustellen. Außerdem ist es möglich, verschiedene Dosierungsformen mittels einer einzigen Basiszubereitung zur Verfügung zu stellen. Dies vereinfacht Herstellung und Lagerung der Zubereitungen und senkt die Kosten.

Die nachfolgenden Beispiele erläutern die Erfindung näher, ohne den Umfang der Erfindung zu beschränken.

### Beispiel 1

In der Tabelle I ist eine Rezeptur für eine erfindungsgemäße Zusammensetzung eines oralen Schilddrüsentherapeutikums angegeben. Dabei beträgt das Verhältnis in Gew.-% von
Wirkstoff: Antioxidationsmittel: Adsorptionsmittel 1 : 7 : 32.

**Tabelle I**

| **Komponente** | **Gew.-%** |
|---|---|
| L-Thyroxin Na x 5 H₂O | 0,1 |
| Mikrokristalline Cellulose | 19,2 |
| Prägelatinierte Stärke | 44,8 |
| Maisstärke | 30,0 |
| L-Cystein x HCl x H₂O | 0,7 |
| Magnesiumoxid | 3,2 |
| Natriumcarbonat | 2,0 |
| | **100,0** |

### Beispiel 2

In der Tabelle II ist eine Rezeptur für eine erfindungsgemäße Zusammensetzung eines oralen Schilddrüsentherapeutikums angegeben. Dabei beträgt das Verhältnis in Gew.-% von
Wirkstoff: Antioxidationsmittel: Adsorptionsmittel 1 : 5 : 47.

**Tabelle II**

| **Komponente** | **Gew.-%** |
|---|---|
| L-Thyroxin Na x 5 H₂O | 0,1 |
| Mikrokristalline Cellulose | 58,5 |
| Maisstärke | 34,2 |
| L-Cystein x HCl x H₂O | 0,5 |
| Magnesiumoxid | 4,7 |
| Talkum | 2,0 |
| | **100,0** |

### Beispiel 3

In der Tabelle III ist eine Rezeptur für eine erfindungsgemäße Zusammensetzung eines oralen Schilddrüsentherapeutikums angegeben. Dabei beträgt das Verhältnis in Gew.-% von
Wirkstoff: Antioxidationsmittel: Adsorptionsmittel 1 : 5 : 32.

**Tabelle III**

| **Komponente** | **Gew.-%** |
|---|---|
| L-Thyroxin Na x 5 H₂O | 0,1 |
| Mikrokristalline Cellulose | 19,2 |
| Prägelatinierte Stärke | 45,0 |
| Maisstärke | 30,0 |
| L-Cystein x HCl x H₂O | 0,5 |
| Magnesiumoxid | 3,2 |
| Talkum | 2,0 |
| | **100,0** |

### Beispiel 4

### Stabilität der erfindungsgemäßen oralen Schilddrüsentherapeutika

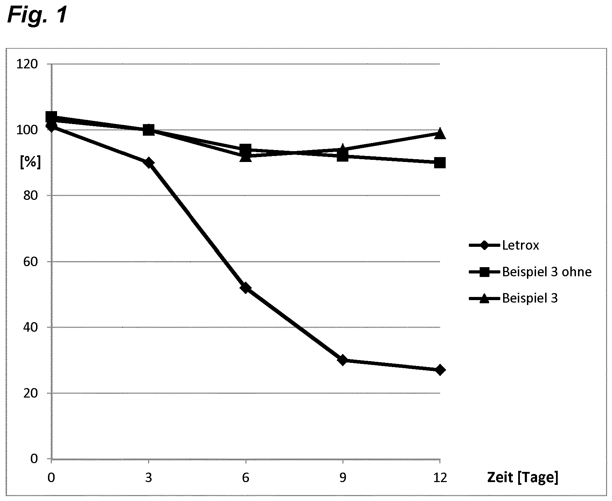

Unter Verwendung der Rezeptur gemäß Beispiel 3 wurden Tabletten mit einer Wirkstoffmenge von 100 µg Levothyroxin-Na hergestellt. Außerdem wurden ebensolche Tabletten ohne das Antioxidationsmittel (Cystein) und ohne das Adsorptionsmittel (Magnesiumdioxid) hergestellt. Diese beiden Zubereitungen wurden dann zusammen mit einem Handelsprodukt (Letrox® 100) einem Stresstest unterzogen. Hierbei wurde eine Lagerung der Zubereitungen über 12 Tage bei einer Temperatur von 70 °C und einer relativen Luftfeuchte von 100% durchgeführt. Der Gehalt an Wirkstoff wurde vor der Lagerung und nach 3, 6, 9 und 12 Tagen Lagerung ermittelt. Die Versuchsergebnisse sind in der Figur 1 dargestellt. Es zeigt sich, dass die Rezeptur gemäß Beispiel 3 (mit Antioxidationsmittel und Adsorptionsmittel) nach 12 Tagen nur eine sehr kleine Verringerung des Wirkstoffgehalts aufwies.

### Beispiel 5

### Herstellung von unterschiedlich dosierten Levothyroxin-Natrium-Tabletten

Unter Verwendung der Rezeptur aus dem Beispiel 1 ist es möglich, allein durch Variation der Tablettenmasse unterschiedlich dosierte Tabletten herzustellen. Hierbei bleibt die prozentuale Zusammensetzung und somit auch das Verhältnis zwischen Wirkstoff (Levothyroxin), Antioxidationsmittel und Adsorptionsmittel sowie Hilfsstoffen unverändert.

Ausgehend von der in Tabelle I des Beispiels 1 angegeben Zusammensetzung der einzelnen Komponenten wird eine Mischung zur Tablettierung hergestellt, welche in 104 mg Mischung 100 µg Wirkstoff enthält. Entsprechend der beim Pressen gewählten Tablettenmasse kann dann die Dosierung des Wirkstoffs eingestellt werden. Die ist der nachfolgenden Übersicht zu entnehmen.

| | |
|---|---|
| Dosierung 25 µg | Tablettenmasse: 26,0 mg |
| Dosierung 50 µg | Tablettenmasse: 52,0 mg |
| Dosierung 75 µg | Tablettenmasse: 78,0 mg |
| **Dosierung 100 µg** | **Tablettenmasse: 104,0 mg** |
| Dosierung 125 µg | Tablettenmasse: 130,0 mg |
| Dosierung 150 µg | Tablettenmasse: 156,0 mg |
| Dosierung 175 µg | Tablettenmasse 182 mg |
| Dosierung 200 µg | Tablettenmasse 208 mg |
| Dosierung 300 µg | Tablettenmasse 312 mg |

### Bei der Verwendung von Bruchrillen kann es jedoch erforderlich sein, die

Tablettengröße zu erhöhen, um eine bessere Zerbrechbarkeit zu erzielen. In diesem Falle wird die Tablettenmasse für die 100 µg Dosierung verdoppelt und beträgt dann 208 mg. Hierbei ist zu beachten, dass das Verhältnis von Wirkstoff zu Antioxidationsmittel zu Adsorptionsmittel nicht verändert wird. Hierzu ist es erforderlich, die Mengen der übrigen Tablettierhilfsstoffe entsprechend zu erhöhen, ohne das erfindungsgemäße Verhältnis von Wirkstoff, Antioxidationsmittel und Adsorptionsmittel zu verändern. Die beim Pressen der Tabletten zu wählende Tablettenmasse ist in der nachfolgenden Übersicht dargestellt.

| | |
|---|---|
| Dosierung 25 µg | Tablettenmasse: 52,0 mg |
| Dosierung 50 µg | Tablettenmasse: 104,0 mg |
| Dosierung 75 µg | Tablettenmasse: 156,0 mg |
| **Dosierung 100 µg** | **Tablettenmasse: 208,0 mg** |
| Dosierung 125 µg | Tablettenmasse: 260,0 mg |
| Dosierung 150 µg | Tablettenmasse: 312,0 mg |

### Beispiel 6

### Ermittlung der Freisetzungsrate

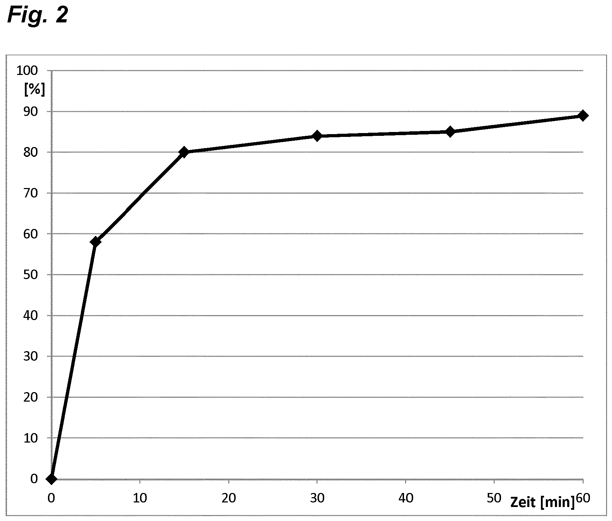

In der Figur 2 ist das Freisetzungsprofil der erfindungsgemäßen Zubereitung gemäß Beispiel 3 gezeigt. Die Tabletten weisen bei einer Masse von 104 mg einen Wirkstoffgehalt von 100 µg auf. Die Untersuchungen wurden in einer Paddle-Apparatur bei 100 min⁻¹ durchgeführt. Das Volumen des Freisetzungsmediums beträgt 500 ml, das Freisetzungsmedium ist Phosphatpuffer mit einem pH-Wert von 7,4.

Nach 15 min sind bereits 80% des Wirkstoffes freigesetzt, nach 60 Minuten mehr als 90%.

Damit ist gezeigt, dass die zur Stabilisierung des Wirkstoffs verwenden Stoffe, nämlich Antioxidationsmittel und Adsorptionsmittel, die Freisetzung und somit auch die Bioverfügbarkeit nicht negativ beeinflussen.

## Patentansprüche

1. Orales Schilddrüsentherapeutikum, enthaltend mindestens einen Wirkstoff in Form eines Schilddrüsenhormons oder dessen pharmazeutisch verträgliche Salze, in Kombination mit mindestens einem Antioxidationsmittel und mindestens einem Adsorptionsmittel, wobei das Verhältnis in Gew.-% von Wirkstoff: Antioxidationsmittel : Adsorptionsmittel 1 : 1 : 5 bis 1 : 50 : 100 beträgt,
**dadurch gekennzeichnet, dass** das Antioxidationsmittel aus schwefelhaltigen, organischen Antioxidationsmitteln ausgewählt ist, die ausgewählt sind aus Cystein, Cystin, Glutathion, Dimercaptopropansulfonsäure sowie deren Salze und aus Mischungen der vorgenannten Mittel, und dass das Adsorptionsmittel aus anorganischen Adsorptionsmitteln ausgewählt ist.

2. Orales Schilddrüsentherapeutikum, gemäß Anspruch 1, **dadurch gekennzeichnet, dass** der Wirkstoff in Form des Schilddrüsenhormons Levothyroxin oder dessen pharmazeutisch verträglicher Salze oder Liothyronin oder dessen pharmazeutisch verträglicher Salze ist oder dass Mischungen aus den genannten Wirkstoffen enthalten sind.

3. Orales Schilddrüsentherapeutikum, gemäß Anspruch 1 oder 2, **dadurch gekennzeichnet, dass** ein weiterer Wirkstoff enthalten ist, wobei der weitere Wirkstoff bevorzugt mindestens ein lodsalz ist, ausgewählt aus Iodiden oder Iodaten der Alkalimetalle, insbesondere Kaliumiodid oder Kaliumiodat, sowie Mischungen der genannten lodsalze.

4. Orales Schilddrüsentherapeutikum, gemäß mindestens einem der voranstehenden Ansprüche, **dadurch gekennzeichnet, dass** weiterhin pharmazeutisch annehmbare Hilfsstoffe, Trägerstoffe und/oder Verdünnungsstoffe enthalten sind.

5. Orales Schilddrüsentherapeutikum, gemäß mindestens einem der voranstehenden Ansprüche , **dadurch gekennzeichnet, dass** das schwefelhaltige, organische Antioxidationsmittel Cystein ist.

6. Orales Schilddrüsentherapeutikum, gemäß Anspruch 5, **dadurch gekennzeichnet, dass** die anorganischen basischen oder oxydischen Adsorptionsmittel ausgewählt sind aus Kieselgelen, Zeolithen, Erdalkalimetalloxiden, Alkalicarbonaten, Alkalihydrogencarbonaten, Erdalkalicarbonaten, Erdalkalihydrogencarbonaten, Erdalkalisilikaten sowie aus Mischungen der vorgenannten Adsorptionsmittel.

7. Orales Schilddrüsentherapeutikum, gemäß Anspruch 6, **dadurch gekennzeichnet, dass** das Erdalkalimetalloxid Magnesiumoxid und/oder das Erdalkalisilikat Talkum und/oder das Alkalicarbonat Natriumcarbonat ist.

8. Orales Schilddrüsentherapeutikum, gemäß mindestens einem der voranstehenden Ansprüche, **dadurch gekennzeichnet, dass** das Verhältnis in Gew.-% von Wirkstoff: Antioxidationsmittel : Adsorptionsmittel 1 : 5 : 5 bis 1 : 10 : 40 beträgt.

9. Orales Schilddrüsentherapeutikum, gemäß mindestens einem der voranstehenden Ansprüche, **dadurch gekennzeichnet, dass** der Wirkstoffgehalt 0,01 bis 0,5 Gew.-% beträgt.

10. Orales Schilddrüsentherapeutikum, gemäß mindestens einem der voranstehenden Ansprüche, **dadurch gekennzeichnet, dass** der Wirkstoffgehalt einer Einzeldosierung 25 µg bis 150 µg beträgt.

11. Orales Schilddrüsentherapeutikum, gemäß einem der voranstehenden Ansprüche, in Form einer einfach oder mehrfach teilbaren Tablette.

12. Orales Schilddrüsentherapeutikum, gemäß Anspruch 11, **dadurch gekennzeichnet, dass** die teilbare Tablette eine Bruchrille, mehrere parallel zueinander angeordnete Bruchrillen oder eine Kreuzbruchrille aufweist.

## Claims

1. An oral thyroid therapeutic agent, containing at least one active ingredient in the form of a thyroid hormone or its pharmaceutically acceptable salts in combination with at least one antioxidant and at least one adsorbent, wherein the ratio of active ingredient: antioxidant:
adsorbent in wt.-% is 1 : 1: 5 to 1 : 50 : 100,
**characterized in that** the antioxidant is selected from sulphur-containing, organic antioxidants, wherein the sulphur-containing, organic antioxidants are selected from cysteine, cystine, glutathione,
dimercaptopropane sulphonic acid and their salts and from mixtures of the aforementioned agents and that the adsorbent is selected from anorganic adsorbents.

2. The oral thyroid therapeutic agent, according to claim 1, **characterized in that** the active ingredient is in the form of the thyroid hormone levothyroxine or its pharmaceutically acceptable salts or liothyronine or its pharmaceutically acceptable salts or that mixtures of said active ingredients are included.

3. The oral thyroid therapeutic agent, according to claim 1 or 2, **characterized in that** a further active ingredient is contained, wherein the further active ingredient is preferably at least one iodine salt selected from iodides or iodates of the alkali metals, in particular potassium iodide or potassium iodate, and mixtures of said iodine salts.

4. The oral thyroid therapeutic agent, according to least one of the preceding claims, **characterized in that** furthermore pharmaceutically acceptable excipients, carriers and/or diluents are included.

5. The oral thyroid therapeutic agent, according to at least one of the preceding claims, **characterized in that** the sulphur-containing organic antioxidant is cysteine.

6. The oral thyroid therapeutic agent, according to claim 5, **characterized in that** the inorganic basic or oxidic adsorbents are selected from silica gels, zeolites, alkaline earth metal oxides, alkali carbonates, alkaline bicarbonates, alkaline earth metal carbonates, alkaline earth bicarbonates, alkaline earth silicates and mixtures of the aforementioned adsorbents.

7. The oral thyroid therapeutic agent, according to claim 6, **characterized in that** the alkaline earth metal oxide is magnesium oxide and/or the alkaline earth metal silicate is talc and/or the alkali carbonate is sodium carbonate.

8. The oral thyroid therapeutic agent, according to at least one of the preceding claims, **characterized in that** the ratio of active ingredient: antioxidant: adsorbent in wt.-% is 1 : 5 : 5 to 1 : 10 : 40.

9. The oral thyroid therapeutic agent, according to at least one of the preceding claims, **characterized in that** the active ingredient content is 0.01 to 0.5 wt.-%.

10. The oral thyroid therapeutic agent, according to least one of the preceding claims, **characterized in that** the active ingredient content of a single dosage is 25 µg to 150 µg.

11. The oral thyroid therapeutic agent, according to at least one of the preceding claims, in the form of tablet which can be divided once or several times.

12. The oral thyroid therapeutic agent, according to claim 11, **characterized in that** the divisible tablet has a breaking groove, multiple breaking grooves, arranged parallel to one another, or a cross-breaking groove.

## Revendications

1. Agent thérapeutique oral pour la thyroïde contenant au moins un principe actif sous la forme d'une hormone thyroïdienne ou les sels pharmaceutiquement acceptables de celle-ci, en combinaison avec au moins un antioxydant et au moins un adsorbant, le rapport en % en poids principe actif : antioxydant : adsorbant étant de 1 :1 :5 à 1 :50 :100, **caractérisé en ce que** l'antioxydant est choisi parmi les antioxydants organiques contenant du soufre choisis parmi la cystéine, la cystine, le glutathion, l'acide dimercaptopropane sulfonique et leurs sels, et parmi les mélanges des agents susmentionnés, et **en ce que** l'adsorbant est choisi parmi les adsorbants inorganiques.

2. Agent thérapeutique oral pour la thyroïde selon la revendication 1, **caractérisé en ce que** le principe actif sous la forme d'une hormone thyroïdienne est la lévothyroxine ou les sels pharmaceutiquement acceptables de celle-ci ou la liothyronine ou les sels pharmaceutiquement acceptables de celle-ci ou **en ce que** des mélanges desdits principes actifs sont inclus.

3. Agent thérapeutique oral pour la thyroïde selon la revendication 1 ou la revendication 2, **caractérisé en ce qu'**il contient un autre principe actif, l'autre principe actif étant de préférence au moins un sel d'iode choisi parmi les iodures ou les iodates des métaux alcalins, en particulier l'iodure de potassium ou l'iodate de potassium, et les mélanges desdits sels d'iode.

4. Agent thérapeutique oral pour la thyroïde selon au moins une des revendications précédentes, **caractérisé en ce qu'**il contient également des excipients, supports et/ou diluants pharmaceutiquement acceptables.

5. Agent thérapeutique oral pour la thyroïde selon au moins une des revendications précédentes, **caractérisé en ce que** l'antioxydant organique contenant du soufre est la cystéine.

6. Agent thérapeutique oral pour la thyroïde selon la revendication 5, **caractérisé en ce que** les adsorbants inorganiques basiques ou oxydants sont choisis parmi les gels de silice, les zéolites, les oxydes de métaux alcalino-terreux, les carbonates alcalins, les hydrogénocarbonates alcalins, les carbonates alcalino-terreux, les hydrogénocarbonates alcalino-terreux, les silicates alcalino-terreux et les mélanges des adsorbants susmentionnés.

7. Agent thérapeutique oral pour la thyroïde selon la revendication 6, **caractérisé en ce que** l'oxyde de métal alcalino-terreux est l'oxyde de magnésium et/ou le silicate alcalino-terreux est le talc et/ou le carbonate alcalin est le carbonate de sodium.

8. Agent thérapeutique oral pour la thyroïde selon au moins une des revendications précédentes, **caractérisé en ce que** le rapport en % en poids principe actif : antioxydant : adsorbant est de 1 :5 :5 à 1 :10 :40.

9. Agent thérapeutique oral pour la thyroïde selon au moins une des revendications précédentes, **caractérisé en ce que** la teneur en principe actif est de 0,01 à 0,5 % en poids.

10. Agent thérapeutique oral pour la thyroïde selon au moins une des revendications précédentes, **caractérisé en ce que** la teneur en principe actif d'une dose unique est de 25 µg à 150 µg.

11. Agent thérapeutique oral pour la thyroïde selon l'une des revendications précédentes, sous la forme d'un comprimé unique ou d'un comprimé multiple apte à être divisé.

12. Agent thérapeutique oral pour la thyroïde selon la revendication 11, **caractérisé en ce que** le comprimé apte à être divisé présente une rainure de fractionnement, plusieurs rainures de fractionnement disposées parallèlement les unes aux autres ou une rainure de fractionnement transversale.
